(19) Europäisches Patentamt
European Patent Office
Office européen des brevets

(11) Veröffentlichungsnummer: **0 069 049**
**B1**

(12) # EUROPÄISCHE PATENTSCHRIFT

(45) Veröffentlichungstag der Patentschrift: **21.05.86**

(51) Int. Cl.⁴: **C 07 C 177/00**, C 07 C 35/06, A 61 K 31/557

(21) Anmeldenummer: **82730084.9**

(22) Anmeldetag: **23.06.82**

(54) **Delta 8,9-Prostaglandinderivate, Verfahren zu ihrer Herstellung und ihre Verwendung als Arzneimittel.**

(30) Priorität: **24.06.81 DE 3125271**

(43) Veröffentlichungstag der Anmeldung:
**05.01.83 Patentblatt 83/1**

(45) Bekanntmachung des Hinweises auf die Patenterteilung:
**21.05.86 Patentblatt 86/21**

(84) Benannte Vertragsstaaten:
**AT BE CH DE FR GB IT LI LU NL SE**

(56) Entgegenhaltungen:
**FR - A - 2 290 192**

**IL FARMACO, Band 31, Nr. 9, September 1976, Seiten 649-654 A. ERMILI et al.: "Ricerche chimiche e farmacologiche su derivati piranici. Nota VI-2-Dialchilammino-4-oxo-4H-nafto(1,2-b)pirani e derivati"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 23, D-1000 Berlin 28 (DE)**
Erfinder: **Radüchel, Bernd, Dr., Gollanczstrasse 132, D-1000 Berlin 28 (DE)**
Erfinder: **Schwarz, Norbert, Dr., Stubenrauchstrasse 31, D-1000 Berlin 41 (DE)**
Erfinder: **Vorbrüggen, Helmut, Prof., Wilkestrasse 7, D-1000 Berlin 27 (DE)**
Erfinder: **Elger, Walter, Dr., Schorlemer Allee 12b, D-1000 Berlin 33 (DE)**
Erfinder: **Loge, Olaf, Dr., Bekassinen Weg 37, D-1000 Berlin 27 (DE)**
Erfinder: **Town, Michael-Harold, Dr., Buchsbaumweg 3, D-1000 Berlin 47 (DE)**

**EP 0 069 049 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue $\Delta^{8,9}$-Prostaglandinderivate, Verfahren zu ihrer Herstellung sowie ihre Verwendung als Arzneimittel.

Aus dem umfangreichen Stand der Technik der Prostaglandine und ihrerAnaloga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metabolisch abgebaut werden. Alle Strukturveränderungen haben das Ziel, die Wirkungsdauer sowie die Selektivität der Wirksamkeit zu steigern.

$\Delta^{9,10}$-Prostanderivate sind in der französischen Patentanmeldung FR-A-2290 192 beschrieben. In Il Farmaco 31 (1976) 649 wird als ungewolltes, in geringer Ausbeute anfallendes Nebenprodukt einer Acylierungsreaktion eine Substanz erwähnt, deren Struktur "wahrscheinlich" 11,15-Dihydroxy-5,5,13-prostatriensäuremethylester ist; biologische Daten dieser Verbindung werden nicht beschrieben.

Es wurde nun gefunden, dass die neuen $\Delta^{8,9}$-Prostaglandinderivate eine ausgeprägte Wirkungsspezifität, bessere Wirksamkeit, längere Wirkungsdauer als natürliche Prostaglandine besitzen und besonders für die orale Applikation geeignet sind. Die neuen $\Delta^{8,9}$-Prostaglandine sind chemisch stabil.

Die Erfindung betrifft $\Delta^{8,9}$-Prostanderivate der allgemeinen Formel I

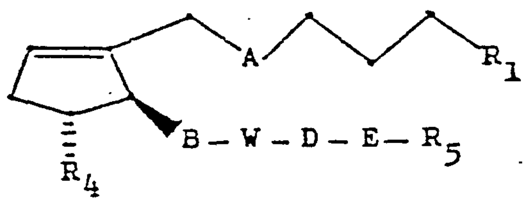

(I),

worin

$R_1$ den Rest $CH_2OH$ oder

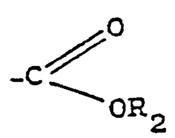

mit $R_2$ in der Bedeutung eines Wasserstoffatoms oder einer Alkylgruppe mit 1-4 C-Atomen, oder $R_1$ den Rest

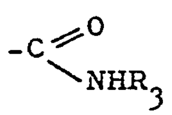

mit $R_3$ in der Bedeutung eines $C_{1-10}$-Alkanoyl- oder Methansulfonyl- oder Isopropansulfonylrestes oder des Restes $R_2$,

A eine $-CH_2-CH_2-$ oder cis-$CH=CH$-Gruppe,

B eine $-CH_2-CH_2-$, oder trans-$CH=CH$- oder eine $-C\equiv C$-Gruppe,

W eine freie oder funktionell abgewandelte Hydroxymethylengruppe oder eine freie oder funktionell abgewandelte

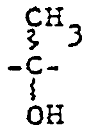

Gruppe, wobei die OH-Gruppe α- oder ß-ständig sein kann,

D und E gemeinsam eine direkte Bindung oder

D eine geradkettige oder verzweigtkettige Alkylengruppe mit 1-10 C-atomen, die gegebenenfalls durch Fluoratome substituiert sein kann,

E ein Sauerstoffatom oder Schwefelatom oder eine direkte Bindung oder eine -C≡C-Bindung oder eine -$CR_6=R_7$-Gruppe darstellt, wobei $R_6$ und $R_7$ sich unterscheiden und ein Wasserstoffatom oder ein Chloratom oder eine Alkylgruppe mit 1-4 C-Atomen bedeuten,

$R_4$ eine freie oder funktionell abgewandelte Hydroxygruppe,

$R_5$ eine gerad- oder verzweigtkettige gesättigte oder ungesättigte Alkylgruppe mit 1-6 C-Atomen, eine Benzylgruppe oder, wenn E ein Sauerstoffatom bedeutet, eine Phenylgruppe und, falls $R_2$ die Bedeutung eines Wasserstoffatoms hat, deren Salze mit physiologisch verträglichen Basen bedeuten, wobei die Gruppierung -$DER_5$ nicht den Rest n-Pentyl darstellt, wenn W für eine freie Hydroxymethylengruppe steht und gleichzeits B trans. CH=CH-, $R_4$ OH, A cis-CH=CH- und $R_1$ COOH bedeuten.

Als Alkylgruppen $R_2$ sind gerade oder verzweigte Alkylgruppen mit 1-4 C-Atomen zu betrachten, wie beispielsweise Methyl, Athyl, propyl, Butyl, Isobutyl, tert.-Butyl.

Als Säurerest $R_3$ kommen physiologisch verträgliche Säurereste in Frage. Die Säuren sind organische Carbonsäuren mit 1-10 Kohlenstoffatomen, die der aliphatischen Reihe angehören sowie Methansulfon- oder Isopropansulfonsäure. Diese Carbonsäuren sind gesättigt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Onanthsäure, Caprylsäure, Pelargonsäure, Caprinsäure, Trimethylessigsäure, Diäthylessigsäure, tert.-Butylessigsäure.

Die Hydroxygruppen in W und $R_4$ können funktionell abgewandelt sein beispielsweise durch Verätherung oder Veresterung, wobei auch die abgewandelte Hydroxygruppe in W α- oder ß-ständig sein kann.

Als Äther- und Acylreste kommen die dem Fachmann bekannten Reste in Betracht. Bevorzugt sind leicht abspaltbare Ätherreste, wie beispielsweise der Tetrahydropyranyl-, Tetrahydrofuranyl-, α-Äthoxyäthyl-, Trimethylsilyl-, Dimethylsilyl-, tert.-Butyl-silyl- und Tribenzyl-silylrest. Als Acylreste kommen die gleichen wie für $R_3$ genannt in Frage, namentlich genannt seien beispielsweise Acetyl, Propionyl, Butyryl und Benzoyl.

Als Alkylgruppen $R_5$ kommen gerad- und verzweigtkettige, gesättigte und ungesättigte Alkylreste mit 1-6 C-Atomen sowie die Benzylgruppe in Frage. Beispielsweise genannt seien Methyl-, Athyl-, Propyl-, Butyl-, Isobutyl-, tert.-Butyl-, pentyl-, Hexyl-, Butenyl-, Isobutenyl-, Propenyl-, Pentenylgruppen.

Wenn E ein Sauerstoffatom bedeutet, kann $R_5$ auch für eine Phenylgruppe stehen.

Als Alkylengruppe D kommen geradkettige oder verzweigtkettiget Alkylenreste mit 1-10, insbesondere 1-5 C-Atomen, in Frage, die gegebenenfalls durch Fluoratome substituiert sein können. Beispielsweise seien genannt: Methylen, Fluormethylen, Difluormethylen, Äthylen, 1,2-Propylen, Äthyläthylen, Trimethylen, Tetramethylen, Pentamethylen, 1.1-Difluoräthylen, 1-Fluoräthylen, 1-Methyltetramethylen, 1-Methyl-tri-methylen.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Naliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Als Alkylgruppen $R_6$ und $R_7$ kommen gerad- und verzweigtkettige gesättigte Alkylreste mit 1-4 C-Atomen in Frage, wie sie bereits für $R_2$ genannt wurden.

Die Erfindung betrifft ferner ein Verfahren zur Herstellung der erfindungsgemässen $\Delta^{8,9}$-Prostanderivate der allgemeinen Formel I, dadurch gekennzeichnet, dass man in an sich bekannter Weise eine Verbindung der allgemeinen Formel II,

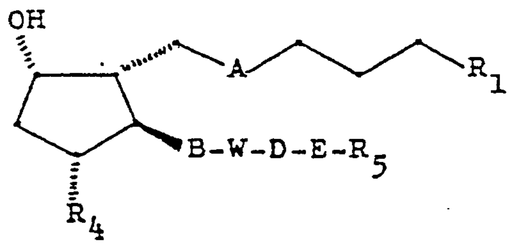

(II)

worin $R_1$ die Bedeutung der reste

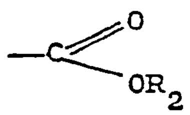

oder

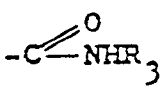

aufweist, A,B,D,E und $R_5$ die obengenannte Bedeutung haben und freie OH-Gruppen in $R_4$ und W geschützt sind, über einen intermediären 9-Sulfonsäureester mit Fluorid als Base umsetzt und gegebenenfalls anschließend in beliebiger Reihenfolge geschützte Hydroxygruppen freisetzt und/oder freie Hydroxygruppen verestert oder veräthert und/oder Doppelbindungen hydriert, und/oder eine veresterte Carboxylgruppe ($R_1$ =

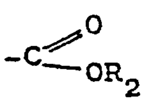 )

verseift und/oder eine Carboxylgruppe ($R_2$ = H) verestert und/oder eine freie Carboxylgruppe ($R_2$ = H) in ein Amid ($R_i$ =

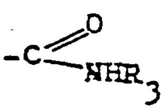 )

überführt und/oder eine freie oder veresterte Carboxylgruppe

($R_1$ = 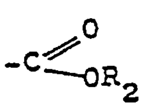 )

reduziert und/oder eine frei Carboxylgruppe in ein Salz überführt

Die Umsetzung der Verbindungen der allgemeinen Formel II zu einem 9-Sulfonsäureester erfolgt in an sich bekannter Weise mit einem Alkyl- oder Arylsulfonylchlorid bzw. Alkyl- oder Arylsulfonsäureanhydrid in Gegenwart eines Amins wie beispielsweise Pyridin, Triäthylamin oder DMAP bei Temperaturen zwischen − 60°C und + 100° C, vorzugsweise − 20°C bis + 50°C. Die Eliminierung des 9-Sulfonats erfolgt mit Fluorid als Base, vorzugsweise Tetrabutylammoniumfluorid in einem inerten Lösungsmittel wie beispielsweise Dimethylformamid, Dimethylacetamid, Hexamethylphosphorsäuretriamid, Dimethylsulfoxid, Dimethoxyäthan, Tetrahydrofuran usw. bei Temperaturen zwischen 0°C und 100°C, vorzugsweise 20°C bis 80 C.

Die Reduktion zu den Verbindungen der allgemeinen Formel I mit $R_1$ in der Bedeutung einer $-CH_2OH$-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diäthyläther, Tetrahydrofuran, Dimethoxyäthan, Toluol usw. in Frage. Die Reduktion wird bei Temperaturen von -30°C bis zur

Siedetemperatur des verwendeten Lösungsmittels, vorzugsweise 0°C bis 30°C vorgenommen.

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wässrigen Lösung einer organischen Säure, wie z.B. Oxalsäure, Essigsäure, Propionsäure u.a., oder in einer wässrigen Lösung einer anorganischen Säure, wie z.B. Salzsäure, durchgeführt. Zur Verbesserung der Löslichkeit wird zweckmässigerweise ein mit Wasser mischbares inertes organisches Lösungsmittel zugesetzt. Geeignete organische Lösungsmittel sind z.B. Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols.

Als Alkohole kommen aliphatische Alkohole in Betracht, wie z.B. Methanol, Äthanol,Butanol usw., vorzugsweise Methanol. Als Alkalicarbonate und -hydroxyde seien Nalium- und Natriumsalze genannt. Bevorzugt sind die Naliumsalze.

Als Erdalkalicarbonate und -hydroxyde sind beispielsweise geeignet Calciumcarbonat, Calciumhydroxyd und Bariumcarbonat. Die Umsetzung erfolgt bei −10°C bis + 70°C, vorzugsweise bei + 25°C.

Die Einführung der Estergruppe

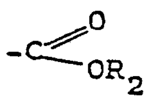

für $R_1$, bei welcher $R_2$ eine Alkylgruppe mit 1-4 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z.B. dadurch, dass man eine Lösung des Diazokohlenwasserstoffes in einem inerten Lösungsmittel, vorzugsweise in Diäthyläther, mit der 1-Carboxyverbindung in dem gleichen oder in einem anderen inerten Lösungsmittel, wie z.B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org.Reactions Bd. 5, Seiten 359-394 (1954)].

Sollen im Primärprodukt enthaltene C=C-Doppelbindungen reduziert werden, erfolgt die Hydrierung nach an sich bekannten Methoden.

Die Hydrierung der 5,6-Doppelbindung wird in an sich bekannter Weise bei tiefen Temperaturen, vorzugsweise bei etwa −20°C, in einer Wasserstoffatmosphäre in Gegenwart eines Edelmetallkatalysators durchgeführt. Als Katalysator ist zum Beispiel 10% Palladium auf Kohle geeignet.

Werden sowohl die 5,6- als auch die 13,14-Doppelbindung hydriert, so arbeitet man bei höherer Temperatur vorzugsweise bei etwa 20°C.

Die Prostaglandinderivate der allgemeinen Formel I mit $R_2$ in der Bedeutung eines Wasserstoffatoms können mit geeigneten Mengen der entsprechenden anorganischen Basen unter Neutralisierung in ein Salz überführt werden. Beispielsweise erhält man beim Lösen der entsprechenden PG-Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes, die in üblicher Weise erfolgt, wird die PG-Säure z.B. in einem geeigneten Lösungsmittel, beispielsweise Äthanol, Aceton, Diäthyläther, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe

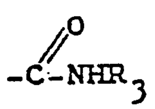

für $R_1$ erfolgt nach den dem Fachmann bekannten Methoden. Die Carbonsäuren der allgemeinen Formel I ($R_2$=H), werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$=H) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen −30°C und + 60°C, vorzugsweise bei 0°C bis 30°C.

Eine weitere Möglichkeit für die Einführung der Amidgruppe

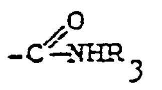

für $R_1$ besteht in der Umsetzung einer 1-Carbonsäure der allgemeinen Formel I ($R_2=H$), in der freie Hydroxygruppen gegebenenfalls intermediär geschützt sind, mit Verbindungen der allgemeinen Formel III,

$O = C = N - R_3$ (III),

worin $R_3$ die obenangegebene Bedeutung hat.

Die Umsetzung der Verbindung der allgemeinen Formel I ($R_2=H$) mit einem Isocyanat der allgemeinen Formel III erfolgt gegebenenfalls unter Zusatz eines tertiären Amins, wie z.B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen $-50°C$ bis $100°C$, vorzugsweise bei 0 bis 30°C,vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmässigerweise von Ausgangsprodukten aus, in denen diese durch vorzugsweise leicht abspaltbare Äther- oder Acylreste intermediär geschützt sind.

Im Vergleich zu PGE-Derivaten zeichnen sich die neuen $\Delta^{8,9}$-Prostaglandine durch grössere Stabilität aus.

Die neuen $\Delta^{5,9}$-Prostanderivate der allgemeinen Formel I sind wertvolle Pharmaka, da sie bei ähnlichem Wirkungsspektrum eine wesentlich verbesserte (höhere Spezifität) und vor allem wesentlich längere Wirkung aufweisen als die entsprechenden natürlichen Prostaglandine.

Die neuen Prostaglandin-Analoga wirken stark luteolytisch, d.h. zur Auslösung einer Luteolyse benötigt man wesentlich geringere Dosierungen als bei den entsprechenden natürlichen Prostaglandinen.

Auch zur Auslösung von Aborten, insbesondere nach oraler oder intravaginaler Applikation, sind wesentlich geringere Mengen der neuen Prostaglandinanaloga im Vergleich zu den natürlichen Prostaglandinen erforderlich.

Bei der Registrierung der isotonischen Uteruskontraktion an der narkotisierten Ratte und am isolierten Rattenuterus zeigt sich, dass die erfindungsgemässen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler-Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw. Ferner eignen sich die erfindungsgemässen Prostaglandin-Derivate zur Cervixdilatation als Vorbereitung für diagnostische oder therapeutische Eingriffe.

Die gute Gewebsspezifität der erfindungsgemässen antifertil wirksamen Substanzen zeigt sich bei der Untersuchung an anderen glattmuskulären Organen, wie beispielsweise am Meerschweinchen-Ileum oder an der isolierten Naninchen-Trachea, wo eine wesentlich geringere Stimulierung zu beobachten ist als durch die natürlichen Prostaglandine. Die erfindungsgemässen Substanzen wirken auch bronchospasmolytisch. Ausserdem bewirken sie eine Abschwellung der Nasenschleimhaut.

Die erfindungsgemässen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese - Inhibitoren) entgegen.

Einige der Verbindungen wirken blutdrucksenkend, regulierend bei Herzrhythmusstörungen und hemmend auf die Plättchenaggregation mit den sich daraus ergebenden Einsatzmöglichkeiten.

Für die medizinische Anwendung können die Wirkstoffe in eine für die Inhalation, für orale, parenterale oder lokale (z.B. vaginale) Applikation geeignete Form überführt werden.

Zur Inhalation werden zweckmässigerweise Arosollösungen hergestellt.

Für die orale Applikation sind beispielsweise Tabletten, Dragees oder Napseln geeignet.

Für die parenterale Verabreichung werden sterile injizierbare, wässrige oder ölige Lösungen benutzt.

Für die vaginale Applikation sind z.B. Zäpfchen geeignet und üblich.

Die Erfindung betrifft damit auch Arzneimittel auf Basis der Verbindungen der allgemeinen Formel I und der üblichen Hilfs- und Trägerstoffe.

Die erfindungsgemässen Wirkstoffe sollen in Verbindung mit den in der Galenik bekannten und üblichen Hilfsstoffen, z.B. zur Herstellung von Präparaten zur Auslösung eines Abortes, zur Zyklussteuerung, zur Einleitung einer Geburt oder zur Behandlung der Hypertonie dienen. Für diesen Zweck aber auch für die übrigen Anwendungen können die Präparate 0,01 - 50 mg der aktiven Verbindung enthalten.

Die folgenden Beispiele sollen die Erfindung näher erläutern, ohne dass damit eine Begrenzung vorgenommen wird.

**Beispiel 1:**

**(5Z,13E)-(11R,15R)-11,15-Dihydroxy-16-phenoxy-17,15,19, 20-tetranor-5,8,13-prostatriensäuremethylester**

Zu einer Lösung von 2,85 g (5Z,13E)-(9S,11R,15R)-9-Hydroxy-11,15-bis-(tetrahydropyran-2-yloxy)-16-phenoxy-17,18,19,20-tetranor-5,13-prostadiensäuremethylester (hergestellt aus der entsprechenden Säure in Methylenchlorid mit 0,5 molarer ätherischer Diazomethanlösung bei 0°C) in 9 ml Pyridin fügt man bei 0°C 1,9 g p-Toluolsulfonsäurechlorid, rührt 16 Stunden bei Eisbadtemperatur und 48 Stunden bei 20°C. Anschliessend

versetzt man mit 6 ml Wasser, rührt 3 Stunden bei 20° C, verdünnt mit Äther, schüttelt nacheinander mit Wasser, 5%iger Schwefelsäure, 5%iger Natriumbicarbonatlösung und Wasser. Man trocknet über Magnesiumsulfat und dampft im Vakuum ein. Dabei erhält man 3,3 g des 9-Tosylats als farbloses Öl.

IR: 2950, 2875, 1734, 1600, 1590, 1496, 1365, 1240, 974/cm

Zu einer Lösung von 3,3 g des vorstehend erhaltenen 9-Tosy-lats in 90 ml abs. Tetrahydrofuran fügt man 6 g Tetrabutylammoniumfluorid und rührt 3 Stunden bei 22° C unter Argon. Anschliessend verdünnt man mit 300 ml Äther, schüttelt dreimal mit Wasser, trocknet über Magnesiumsulfat und dampft im Vakuum ein. Den Rückstand reinigt man durch Chromatographie an Nieselgel. Mit Hexan/Äther (4+1) erhält man 1,8 g (5Z,13E)-(11R,15R)-11,15-bis-(tetrahydropyran-2-yl-oxy)-16-phenoxy-17,18,19,20-tetranor-5,5,13-prostatriensäuremethylester als farbloses Öl.

IR: 2960, 2855, 1730, 1600, 1588, 1495, 970/cm.

Zur Schutzgruppenabspaltung rührt man 1,5 g der vorstehend erhaltenen $\Delta^{8,9}$-Verbindung 16 Stunden bei 22° C mit 60 ml einer Mischung aus Essigsäure, Wasser, Tetrahydrofuran (65+35+10) und dampft anschliessend im Vakuum ein. Den Rückstand reinigt man durch Chromatographie an Nieselgel.

Mit Äther als Elutionsmittel erhält man 0,95 g der Titelverbindung als farbloses Öl.

IR: 3600, 3420 (breit), 2930, 2855, 1730, 1600, 1588, 971/ cm.

**Beispiel 2:**

**(13E)-(11R,15R)-11,15-Dihydroxy-16-phenoxy-17,18,19,20-tetranor-5,13-prostadiensäuremethylester**

In Analogie zu Beispiel 1 erhält man aus 1,35 g (13E)-(9S, 11R,15R)-9-Hydroxy-11,15-bis-(tetrahydropyran-2-yloxy)-16-phenoxy-17,18,19,20-tetranor-13-prostensäuremethylester, 500 mg (13E)-(11R,15R)-11,15-bis-(tetrahydropyran-2-yloxy)-16-phenoxy-17,18,19,20-tetranor-5,13-prostadiensäuremethylester als farbloses Öl.

IR: 2960, 2858, 1730, 1600, 1588, 1496, 972/cm.

Nach Abspaltung der Schutzgruppen gemäss Beispiel 1 erhält man 0,4 g der Titelverbindung als farbloses Öl.

IR: 3610, 3400 (breit), 2930, 2856, 1730, 1600, 1588, 972/cm 1H-NMR($CDCl_3$): δ = 3,68 (3) s

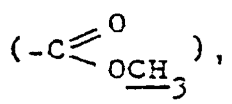

5,36(1)q(I = 4 Hz,H-9), 4,09-4,29(1)m(H-11), 5,63-5,71(2) (H-13,14), 4,44-4,62(1)m(H-15), 3,78-4,04(2)m(H-16), 6,87-7,40(5)m (arom.H).

**Beispiel 3:**

**(5Z,13E)-(11R,15R)-11,15-Dihydroxy-16,16-dimethyl-5,8, 13-prostatriensäuremethylester**

In Analogie zu Beispiel 1 erhält man aus 2,75 g (5Z,13E)-(9S,11R,15R)-11,15-Bis-(tetrahydropyran-2-yloxy)-16,16-dimethyl-9-hydroxy-5,13-prostadiensäuremethylester 1,6 g (5Z,13E)-(11R,15R)-16,16-Dimethyl-11,15-bis-(tetrahydropyran-2-yloxy)-5,8,13-prostatriensäuremethylester als farbloses Öl.

IR: 2960, 2855, 1732, 975/cm.

Nach Abspaltung der Schutzgruppen gemäss Beispiel 1 erhält man 0,5 g der Titelverbindung als farbloses Öl.

IR: 3610, 3400 (breit), 2940, 2550, 1732, 975/cm.

**Beispiel 4:**

**(13E)-(11R,15R)-11,15-Dihydroxy-16,16-dimethyl-8,13-prostadiensäuremethylester**

In Analogie zu Beispiel 1 erhält man aus 300 mg (13E)-(9S,11R,15R)-11,15-Bis-(tetrahydropyran-2-yloxy)-16,16-dimethyl-9-hydroxy-13-prostensäuremethylester 170 mg (13E)-(11R,15R)-16,16-Dimethyl-11,15-bis-(tetrahydropyran-2-yloxy)-8,13-prostadiensäuremethylester als farbloses Öl.

IR: 2955, 2860, 1732, 1495, 975/cm.

Nach Abspaltung der Schutzgruppen gemäss Beispiel 1 erhält man 90 mg der Titelverbindung als farbloses Öl.

IR: 3600, 3410 (breit), 2940, 2855, 1732, 975/cm.

**Beispiel 5:**

**(5Z,13E)-(11R,16RS)-11,15 α-Dihydroxy-16-methyl-5,8,13-prostatriensäuremethylester**

In Analogie zu Beispiel 1 erhält man aus 1,4 g (5Z,13E)-(9S,11R,16RS)-11,15 α-Bis-(tetrahydropyran-2-yloxy)-9-hydroxy-16-methyl-5,13-prostadiensäuremethylester 0,58 g (5Z,13E)-(11R,16RS)-11,15 α-Bis-(tetrahydropyran-2-yloxy)-16-methyl-5,8,13-prostatriensäuremethylester als farbloses Öl.

IR: 2960, 2655, 1730, 1495, 974/cm.

Nach Abspaltung der Schutzgruppen gemäss Beispiel 1 erhält man 0,5 g der Titelverbindung als farbloses Öl.

IR: 3610, 3400 (breit), 2935, 2855, 1730, 974/cm.

**Beispiel 6:**

**(5Z-13E)-(11R,15RS)-11,15-Dihydroxy-15-methyl-5,8,13-prostatriensäuremethylester**

In Analogie zu Beispiel 1 erhält man aus 0,65 g (5Z,13E)-(9S,11R,15RS)-11,15-Bis-(tetrahydropyran-2-yloxy)-9-hy-droxy-15-methyl-5,13-prostadiensäuremethylester 0,35 g (5Z,13E)-(11R,15RS)-11,15-Bis-(tetrahydropyran-2-yloxy)-15-methyl-5,8,13-prostatriensäuremethylester als farbloses Öl.

IR: 1732, 970/cm.

Nach Abspaltung der Schutzgruppen gemäss Beispiel 1 erhält man 0,26 g der Titelverbindung als farbloses Öl.

IR: 3600, 3420 (breit), 2932, 2856, 1732, 970/cm.

**Beispiel 7:**

**(5Z,13E)-(11R,15R,16RS)-11,15-Dihydroxy-16-fluor-5,8,13-prostatriensäuremethylester**
In Analogie zu Beispiel 1 erhält man aus 0,8 g (5Z,13E)-(9S,11R,15R,16RS) -11,15-Bis-(tetrahydropyran-2-yloxy)-9-hydroxy-16-fluor-5,13-prostadiensäuremethylester 0,50 g (5Z,13E)-(11R,15R,16RS)-11,15-Bis-(tetrahydropyran-2-yl-oxy)-16-fluor-5,8,13-prostatriensäuremethylester als farbloses Öl.
IR: 2958, 1735, 976/cm.
Nach Abspaltung der Schutzgruppen gemäss Beispiel 1 erhält man 0,29 g der Titelverbindung als Öl.
IR: 3610, 3400 (breit), 2930, 2857, 1735, 976/cm.

**Beispiel 8:**

**(5Z,13E)-(11R,16RS)-11,15α-Dihydroxy-16,19-dimethyl-5,8,13,18-prostatetranensäuremethylester**
In Analogie zu Beispiel 1 erhält man aus 2,4 g (5Z,13E)-(9S,11R,16RS)-11,15 α-Bis-(tetrahydropyran-2-yloxy)-9-hydroxy-16,19-dimethyl-5,13,18-prostatriensäuremethyl-ester 1,5 g (5Z,13E)-(11R,16RS)-11,15α-Bis-(tetrahydropyran-2-yloxy)-16,19-dimethyl-5,8,13,18-prostatetraensäure-methylester als farbloses Öl.
IR: 2960, 1732, 972/cm.
Nach Abspaltung der Schutzgruppen gemäss Beispiel 1 erhält man 0,78 g der Titelverbindung als farbloses Öl. IR: 3600, 3400 (breit), 2932, 2855, 1732, 972/cm.

**Beispiel 9:**
**(13E)-(11R,16RS)-11,15α-Dihydroxy-16,19-dimethyl-8,13, 15-prostatriensäuremethylester**
In Analogie zu Beispiel 1 erhält man aus 600 mg (13E)-(9S,11R,16RS)-11,15α-Bis-(tetrahydropyran-2-yloxy)-9-hydroxy-16,19-dimethyl-13,18-prostadiensäuremethylester 350 mg (13E)-(11R,16RS)-11,15α-Bis-(tetrahydropyran-2-yloxy)-16,19-dimethyl-8,13,18-prostatriensäuremethyl-ester als farbloses Öl.
IR: 2962, 1731, 972/cm.
Nach Abspaltung der Schutzgruppen gemäss Beispiel 1 erhält man 180 mg der Titelverbindung als farbloses Öl. IR: 3610, 3410 (breit) 2930, 2855, 1731, 972/cm.

**Beispiel 10:**
**(5Z,13E)-(11R,15R)-11,15-Dihydroxy-16,16,19-trimethyl-5,8,13,18-prostatetraensäuremethylester**
In Analogie zu Beispiel 1 erhält man aus 1,35 g (5Z,13E)-(9S,11R,15R)-11,15-Bis-(tetrahydropyran-2-yloxy)-9-hydroxy-16,16,19-trimethyl-5,13,18-prostatriensäuremethyl-ester 0,75 g (5Z,13E)-(11R,15R)-11,15-Bis-(tetrahydropyran-2-yloxy)-16,16,19-trimethyl-5,8,13,18-prostatetra-ensäuremethylester als farbloses Öl.
IR: 2960, 2855, 1732, 974/cm.
Nach Abspaltung der Schutzgruppen gemäss Beispiel 1 erhält man 0,4 g der Titelverbindung als farbloses Öl.
IR: 3600, 3400 (breit), 2942, 2850, 1732, 974/cm.

**Beispiel 11:**
**(13E)-(11R,15R)-11,15-Dihydroxy-16,16,19-trimethyl-8,13, 18-prostatriensäuremethylester**
In Analogie zu Beispiel 1 erhält man aus 600 mg (13E)-(9S,11R,15R)-11,15-Bis-(tetrahydropyran-2-yloxy)-9-hydroxy-16,16,19-trimethyl-13,18-prostadiensäuremethylester 330 mg (13E)-(11R,15R)-11,15-Bis-(tetrahydropyran-2-yl-oxy)-16,16,19-trimethyl-8,13,18-prostatriensäuremethylester als farbloses Öl.
IR: 2962, 2853, 1733, 974/cm.
Nach Abspaltung der Schutzgruppen gemäss Beispiel 1 erhält man 185 mg der Titelverbindung als farbloses Öl. IR: 3610, 3400 (breit), 2940, 2855, 1733, 974/cm.

**Beispiel 12:**
**(5Z,13E)-(11H,15H)-11,15-Dihydroxy-16-phenoxy-17,18,19,20-tetranor-5,8,13-Prostatriensäure**
790 mg des nach Beispiel 1 hergestellten Methylesters rührt man 5 Stunden mit 20 ml einer Lösung aus Kaliumhydroxid in Äthanol und Wasser (Herstellung: Man löst 2 g Naliumhydroxid in 75 ml Äthanol und 25 ml Wasser). Anschliessend säuert man mit 10%iger Zitronensäurelösung auf pH4 an, extrahiert dreimal mit Methylenchlorid, wäscht den organischen Extrakt einmal mit Sole, trocknet über Magnesiumsulfat und dampft im Vakuum ein. Nach Chromatographie des Rückstands an Nieselgel mit Methylenchlorid/ Isopropanol (95+5) als Elutionsmittel erhält man 610 mg der Titelverbindung als farbloses Öl.
IR: 3600, 3400 (breit), 2930, 2858, 1710, 1600, 1587, 1493, 970/cm.

**Beispiel 13: (13E)-(11R,15R)-11,15-Dihydroxy-16-phenoxy-17,18,19,20-tetranor-8,13-prostadiensäure**
In Analogie zu Beispiel 12 erhält man aus 0,3 g des nach Beispiel 2 hergestellten Methylesters 0,24 g der Titelverbindung als Öl.
IR: 3600, 3410 (breit), 2935, 2856, 1710, 1600, 1590, 970/cm.

**Beispiel 14:**
**(5Z,13E)-(11R,15R)-11,15-Dihydroxy-16,16-dimethyl-5,8,13-prostatriensäure**
In Analogie zu Beispiel 14 erhält man aus 0,45 g des nach Beispiel 3 hergestellten ethylesters 0,38g der Titelverbindung als farbloses Öl.
IR: 3600, 3400 (breit), 2940, 2850, 1705, 974/cm.

**Beispiel 15:**
**(13E)-(11R,15R)-11,15-Dihydroxy-16,16-dimethyl-8,13-prostadiensäure**
In Analogie zu Beispiel 12 erhält man aus 120 mg des nach Beispiel 4 hergestellten Methylesters 95 mg der Titelverbindung als farbloses Öl.
IR: 3600, 3410 (breit), 2940, 2855, 1705, 974/cm.

**Beispiel 16:**
**(5Z,13E)-(11R,16RS)-11,15α-Dihydroxy-16-methyl-5,8,13-prostatriensäure**
In Analogie zu Beispiel 12 erhält man aus 0,25 g des nach Beispiel 6 hergestellten Methylesters 0,2 g der Titelverbindung als farbloses Öl.
IR: 3600, 3400 (breit), 2935, 2855, 1710, 974/cm

**Beispiel 17:**
**(5Z,13E)-(11R,15RS)-11,15-Dihydroxy-15-methyl-5,8,13-prostatriensäure**
In Analogie zu Beispiel 14 erhält man aus 0,2 g des nach Beispiel 6 hergestellten Methylesters 0,17 g der Titelverbindung als farbloses Öl.
IR: 3600, 3420 (breit), 2935, 2855, 1710, 972/cm.

**Beispiel 18:**
**(5Z,13E)-(11R,15R,16RS)-11,15-Dihydroxy-16-fluor-5,8,13-prostatriensäure**
In Analogie zu Beispiel 12 erhält man aus 0,4 g des nach Beispiel 7 hergestellten Methylesters 0,3 g der Titelverbindung als farbloses Öl.
IR: 3610, 3400 (breit), 2930, 2855, 1709, 976/cm.

**Beispiel 19:**
**(5Z,13E)-(11R,16RS)-11,15α-Dihydroxy-16,19-dimethyl-5,8, 13,15-prostatetraensäure**
In Analogie zu Beispiel 12 erhält man aus 0,7 g des naαh Beispiel 8 hergestellten Methylesters 0,6 g der Titelverbindung als farbloses Öl.
IR: 3600, 3400 (breit), 2935, 2855, 1710, 972/cm.

**Beispiel 20:**
**(13E)-(11R,16RS)-11,15α-Dihydroxy-16,19-dimethyl-8,13,18-prostatriensäure**
In Analogie zu Beispiel 12 erhält man aus 0,45 g des nach Beispiel 9 hergestellten Methylesters 0,38 g der Titelverbindung als farbloses Öl.
IR: 3600, 3410 (breit), 2930, 2855, 1710, 972/cm.

**Beispiel 21:**
**(5Z,13E)-(11R,15R)-11,15-Dihydroxy-16,16,19-trimethyl-5,8,13,18-prostatetraensäure**
In Analogie zu Beispiel 14 erhält man aus 0,21 g des nach Beispiel 10 hergestellten Methylesters 0,17 g der Titelverbindung als farbloses Öl.
IR: 3600, 3410 (breit), 2940, 2850, 1709, 974/cm.

**Beispiel 22:**
**(13E)-(11R,15R)-11,15-Dihydroxy-16,16,19-trimethyl-8,13,18-prostatriensäure**
In Analogie zu Beispiel 12 erhält man aus 130 mg des nach Beispiel 11 hergestellten Methylesters 110 mg der Titelverbindung als farbloses Öl.
IR: 3600, 3400 (breit), 2940, 2855, 1708, 974/cm.

**Beispiel 23:**
**(5Z,13E)-(11R,15R)-1,11,15-Trihydroxy-16-phenoxy-17, 18,19,20-tetranor-5,8,13-prostatrien**
Zu einer Lösung von 200 mg des nach Beispiel 1 hergestellten Methylesters in 12 ml Tetrahydrofuran fügt man bei 0°C portionsweise 300 mg Lithiumaluminiumhydrid, rührt 45 Minuten bei 0°C und 20 Minuten bei 20°C unter Argon. Anschliessend zerstört man bei 0°C den Reagenzüberschuss durch tropfenweise Zugabe von Essigester, rührt 5 Minuten, fügt 2 ml Wasser und 60 ml Äther zu und rührt 3 Stunden bei 20°C. Man filtriert, wäscht mit Äther, trocknet das Filtrat mit Magnesiumsulfat und dampft im Vakuum ein. Nach Chromatographie des Rückstandes an Kieselgel mit Essigester/Hexan (4+1) erhält man 165 mg der Titelverbindung als farbloses Öl.
IR: 3600, 3430 (breit), 3000, 2930 2858, 1600, 1588 1495, 972/cm.

**Beispiel 24: (13E)-(11R,15R)-1,11,15-Trihydroxy-16-phenoxy-17,18,19, 20-tetranor-5,13-prostadien**
In Analogie zu Beispiel 23 erhält man aus 180 mg des nach Beispiel 2 hergestellten Methylesters 155 mg der Titelverbindung als farbloses Öl.
IR: 3600, 3440 (breit), 3000, 2930, 2857, 1600, 1588, 1495, 972/cm.

**Beispiel 25: (5Z,13E)-(11R,15R)-1,11,15-Trihydroxy-16,16-dimethyl-5,8, 13-prostatrien**
In Analogie zu Beispiel 23 erhält man aus 250 mg des nach Beispiel 3 hergestellten Methylesters 210 mg der Titelverbindung als farbloses Öl.
IR: 3600, 3415 (breit), 2935, 2556, 974/cm.

**Beispiel 26: (5Z,13E)-(11R,16RS)-16-Methyl-1,11,15α-trihydroxy-5,8,13-prostatrien**
In Analogie zu Beispiel 23 erhält man aus 140 mg des nach Beispiel 5 hergestellten Methylesters 110 mg der Titelverbindung als farbloses Öl.
IR: 3600, 3440 (breit), 2935, 2855, 973/cm.

**Beispiel 27: (5Z,13E)-(11R,16RS)-16,19-Dimethyl-1,11,15α-trihydroxy-5,8,13,18-prostatetraen**
In Analogie zu Beispiel 23 erhält man aus 220 mg des nach Beispiel 8 hergestellten Methylesters 180 mg der Titelverbindung als farbloses Öl.
IR: 3610, 3430 (breit), 2933, 2855, 972/cm.

**Beispiel 28:**
**(13E)-(11R,16RS)-16,19-Dimethyl-1,11,15α-trihydroxy-8, 13,18-prostatrien**
In Analogie zu Beispiel 23 erhält man aus 155 mg des nach Beispiel 9 hergestellten Methylesters 130 mg der Titelverbindung als farbloses Öl.
IR: 3600, 1430 (breit), 2933, 2855, 972/cm.

**Beispiel 29:**

**(5Z,13E)-(11R,15R)-11,15-Dihydroxy-16-phenoxy-17,18,19 20-tetranor-5,8,13-prostatriensäure-methylsulfonamid**

Zu einer Lösung von 200 mg (5Z,13E)-(11R,15R)-11,15-Dihydroxy-16-phenoxy-17,18,19,20-tetranor-5,8,13-prostatriensäure (hergestellt nach Beispiel 12) in 5 ml Dimethylformamid gibt man bei 0°C 90 mg Chlorameisensäureisobutylester und 70 mg Triäthylamin. Nach 30 Minuten gibt man 300 mg des Natriumsalzes von Methylsulfonamid (hergestellt aus Methylsulfonamid und Natriummethylat) und 2 ml Hexamethylphosphorsäuretriamid zu und rührt 5 Stunden bei 20°C. Anschliessend verdünnt man mit Citratpuffer (pH 4), extrahiert mit Essigester, wäscht den Extrakt mit Sole, trocknet über Magnesiumsulfat und dampft im Vakuum ein. Nach Chromatographie des Rückstands an Kieselgel mit Methylenchlorid erhält man 131 mg der Titelverbindung als Öl.

IR: 3600, 3410, 2948, 2860, 1720, 1600, 1588, 976/cm.

**Beispiel 30:**

**(13E)-(11R,15R)-11,15-Dihydroxy-16-phenoxy-17,18,19,20-tetranor-8,13-prostadiensäure-methylsulfonamid**

In Analogie zu Beispiel 29 erhält man aus 150 mg (13E)-(11R,15R)-11,15-Dihydroxy-16-phenoxy-17,18,19,20-tetra-nor-8,13-prostadiensäure (hergestellt nach Beispiel 13) 95 mg der Titelverbindung als Öl.

TR: 3600, 3410, 2952, 2860, 1722, 1600, 1589, 976/cm.

**Beispiei 31:**

**(13E)-(11R,15R)-11,15-Dihydroxy-16,16-dimethyl-8,13-prostadiensäure-methylsulfonamid**

In Analogie zu Beispiel 29 erhält man aus 200 mg (13E)-(11R,15R)-11,15-Dihydroxy-16,16-dimethyl-8,13-prostadiensäure (hergestelit nach Beispiel 15) 115 mg der Titelverbindung als Öl.

IR: 3600, 3405 (breit), 2942, 2855, 1720, 974/cm.

**Beispiel 32:**

**(13E)-(11R,16RS)-11,15α-Dihydroxy-16,19-dimethyl-8,13,18-prostatriensäure-methylsulfonamid**

In Analogie zu Beispiel 29 erhält man aus 200 mg (13E)-(11R,16RS)-11,15α-Dihydroxy-16,19-dimethyl-8,13,18-prostatriensäure (hergestellt nach Beispiel 20) 100 mg der Titelverbindung als farbloses Öl.

IR: 3600, 3410 (breit), 2943, 2860, 1722, 976/cm.

**Beispiel 33:**

**(13E)-(11R,15R)-11,15-Dihydroxy-16-phenoxy-17,18,19,20-tetranor-8,13-prostadiensäure-isopropylsulfonamid**

210 mg (13E)-(11R,15R)-11,15-Dihydroxy-16-phenoxy-17,18, 19,20-tetranor-8,13-prostadiensäure (hergestellt nach Beispiel 13) werden in 5 ml Dimethylformamid gelöst und bei 0°C mit 80 mg Chlorameisensäureisobutylester und 60 mg Triäthylamin versetzt. Nach 30 Minuten werden 200 mg des Natriumsalzes von Isopropylsulfonamid (hergestellt aus Isopropylsulfonamid und Natriummethylat) und 2 ml Hexamethylphosphorsäuretriamid zugefügt und 3 Stunden bei 25°C gerührt. Zur Aufarbeitung gibt man 100 ml Citratpuffer (pH4) zu, extrahiert mehrmals mit Essigester wäscht die organische Phase mit Sole, trocknet über Magnesiumsulfat und dampft im Vakuum ein. Nach Chromatographie des Rückstandes an Rieselgel mit Methylenchlorid erhält man 120 mg der Titelverbindung als Öl.

IR: 3600, 3400, 2955, 2866, 1725, 1601, 1588, 1125, 976/cm.

**Beispiel 34:**

**(13E)-(11R,15R)-11,15-Dihydroxy-16-phenoxy-17,18,19,20-tetranor-8,13-prostadiensäure-acetylamid**

Eine Lösung von 557 mg (13E)-(11R,15R)-11,15-Bis-(tetra-hydropyran-2-yloxy)-16-phenoxy-17,18,19,20-tetranor-8,13-prostadiensäuremethylester (hergestellt nach Beispiel 2) in 10 ml Methanol wird mit 2 ml 2N Natronlauge 6 Stunden unter Argon gerührt. Man engt im Vakuum ein, verdünnt mit 50 ml Sole, säuert mit Citronensäure auf pH 4,5 an und extrahiert einige Male mit Essigester. Der organische Extrakt wird mit Sole gewaschen, über Magnesiumsulfat getrocknet und im Vakuum eingedampft. Man erhält als Rückstand 540 mg (13E)-(11R,15R)-11,15-Bis-(tetrahydropy-ran-2-yloxy)-16-phenoxy-17,18,19,20-tetranor-8,13-prostadiensäure.

Zur Bildung des Acetylamids löst man die Säure in 15 ml Acetonitril, versetzt mit 130 mg Triäthylamin und tropft bei 0°C eine Lösung von 102 mg Acetylisocyanat in 10 ml Acetonitril zu. Man rührt noch 2 Stunden bei 20°C, verdünnt mit 100 ml Citratpuffer (pH4), extrahiert mehrmals mit Äther, wäscht die organische Phase mit Sole, trocknet über Magnesiumsulfat und dampft im Vakuum ein. Zur Abspaltung der Schutzgruppen rührt man den Rückstand mit 15 ml Eisessig/Wasser/ Tetrahydrofuran (65/35/10) 4 Stunden bei 40°C und dampft dann im Vakuum zur Trockne. Der Rückstand wird an Kieselgel mit Methylenchlorid unter Zusatz von 0,1 bis 0,5% Isopropylalkohol chromatographiert. Man erhält 200 mg der Titelverbindung als Öl.

IR: 3600, 3405, 2948, 2860, 1710, 1600, 1588, 978/cm.

**Beispiel 35:**

**(13E)-(11R,15R)-11,15-Dihydroxy-16-phenoxy-17,18,19,20-tetranor-8,13-prostadiensäure-amid**

Zu einer Lösung von 210 mg (13E)-(11R,15R)-11,15-Dihydroxy-16-phenoxy-17,18,19,20-tetranor-8,13-prostadiensäure (hergestellt nach Beispiel 13) in 5 ml Tetrahydrofuran gibt man 80 mg Chlorameisensäureisobutylester und 60 mg Triäthylamin und rührt 1 Stunde bei 0°C. Anschliessend wird bei 0°C 10 Minuten Ammoniakgas eingeleitet, dann I Stunde bei 25°C belassen. Zur Aufarbeitung verdünnt man mit Wasser, extrahiert mehrmals mit Methylenchlorid, schüttelt die Extrakte mit Sole, trocknet über Magnesiumsulfat und dampft im Vakuum ein. Zur Reinigung wird an Kieselgel mit Methylenchlorid/1% Isopropylalkohol chromatographiert. Man erhält 130 mg der Titelverbindung als Öl.

IR: 3600, 3540, 3410, 2960, 2858, 1665, 1600, 1585, 974/cm.

**Beispiel 36:**

**(13E)-(11R,16RS)-11,15α-Dihydroxy-16,19-dimethyl-8,13,18-prostatriensäure-amid**

In Analogie zu Beispiel 35 erhält man aus 200 mg (13E)-(11R,16RS)-11,15α-Dihydroxy-16,19-dimethyl-8,13,18-prostatriensäure (hergestellt nach Beispiel 20) 110 mg der Titelverbindung als Öl.

IR: 3600, 3450, 2962, 8548, 1668, 978/cm,

**Beispiel 37:**

**(13E)-(11R,15R)-11,15-Dihydroxy-16-phenoxy-17,18,19,20-tetranor-8,13-prostadiensäure-tris(hydroxymethyl)aminomethansalz**

Zu einer Lösung von 390 mg (13E)-(11R,15R)-11,15-Dihydroxy-16-phenoxy-17,18,19,20-tetranor-8,13-prostadiensäure (hergestellt nach Beispiel 13) in 70 ml Acetonitril gibt man bei 65°C eine Lösung von 122 mg Tris-(hydroxymethyl)-aminomethan in 0,4 ml Wasser. Man lässt unter Rühren auf 20°C abkühlen, dekantiert vom Lösungsmittel und trocknet den Rückstand im Vakuum. Man erhält 320 mg der Titelverbindung als wachsartige Masse.

**Patentansprüche**

1. $\Delta^{8,9}$-Prostanderivate der allgemeinen Formel I

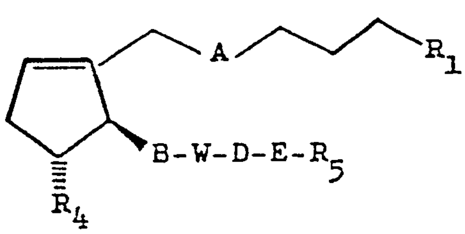

(I),

worin

$R_1$ den Rest-$CH_2OH$ oder

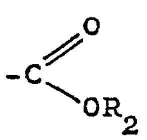

mit $R_2$ in der Bedeutung eines Wasserstoffatoms oder einer $C_{1\text{-}4}$-Alkylgruppe, oder $R_1$ den Rest

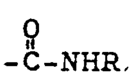

mit $R_3$ in der Bedeutung eines $C_{1\text{-}10}$-Alkanoyl- oder Methansulfonyl- oder Isopropansulfonylrestes oder des Restes $R_2$,

A eine -$CH_2$-$CH_2$- oder cis-CH=CH-Gruppe,

B eine -$CH_2$-$CH_2$-, oder trans-CH=CH- oder eine -C≡C-Gruppe,

W eine freie oder funktionell abgewandelte Hydroxymethylengruppe oder eine freie oder funktionell abgewandelte

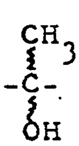

Gruppe, wobei die OH-Gruppe α- oder ß-ständig sein kann,

D und E gemeinsam eine direkte Bindung oder

D eine geradkettige oder verzweigtkettige Alkylengruppe mit 1-10 C-Atomen die gegebenenfalls durch Fluoratome substituiert sein kann,

E ein Sauerstoffatom oder Schwefelatom oder eine direkte Bindung oder eine $-C\equiv C$-Bindung oder eine $-CR_6=CR_7$-Gruppe darstellt, wobei $R_6$ und $R_7$ sich unterscheiden und ein Wasserstoffatom oder ein Chloratom oder eine Alkylgruppe mit 1-4 C-Atomen bedeuten,

$R_4$ eine freie oder funktionell abgewandelte Hydroxygruppe,

$R_5$ eine gerad- oder verzweigtkettige, gesättigte oder ungesättigte Alkylgruppe mit 1-6 C-Atomen, eine Benzylgruppe oder, wenn E ein Sauerstoffatom bedeutet, eine Phenylgruppe; und, falls $R_2$ die Bedeutung eines Wasserstoffatoms hat, deren Salze mit physiologisch vertraglichen Basen bedeuten, wobei die Gruppierung $-DER_5$ nicht den Rest n-Pentyl darstellt, wenn W für eine freie Hydroxymethylengruppe steht und gleichzeitig B trans. $CH=CH$-, $R_4$ OH, A cis-$CH=CH$und $R_1$ COOH bedeuten.

2. Verfahren zur Herstellung der erfindungsgemäßen $\Delta^{8,9}$-Prostanderivate der allgemeinen Formel I, dadurch gekennzeichnet, daß man in an sich bekannter Weise eine Verbindung der allgemeinen Formel II

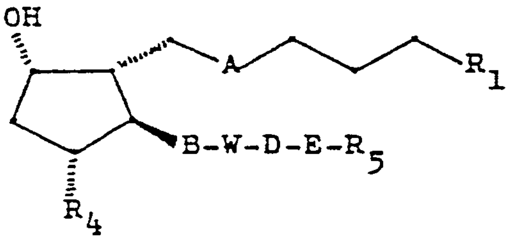

(II) ,

worin

$R_1$ die Bedeutung der Reste

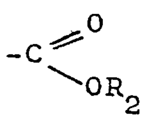

oder

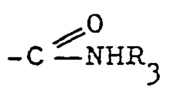

aufweist, A, B, D. E und $R_5$ die obengenannte Bedeutung haben und freie OH-Gruppen in $R_4$ und W geschützt sind, über einen intermediären 9-Sulfonsäureester mit Fluorid als Base umsetzt und gegebenenfalls anschließend in beliebiger Reihenfolge geschützte Hydroxygruppen freisetzt und/oder freie Hydroxygruppen verestert oder veräthert und/oder Doppelbindungen hydriert, und/oder eine veresterte Carboxylgruppe verseift und/oder eine Carboxylgruppe verestert und/oder eine freie Carboxylgruppe in ein Amid überführt und/oder eine freie oder veresterte Carboxylgruppe reduziert und/oder eine freie Carboxylgruppe in ein Salz überführt.

3. Arzneimittel, bestehend aus einer oder mehreren Verbindungen gemäß Anspruch 1 und üblichen Hilfs- und Trägerstoffen.

4. (13E)-(11R,15R)-11,15-Dihydroxy-16-phenoxy-17,18,19,20-tetranor-8,13-prostadiensäure.

5. (5Z,13E)-(11R,15R)-11,15-Dihydroxy-16,16-dimethyl-5,8,13-prostatriensäure.

6. (13E)-(11R,15R)-1,11,15-Trihydroxy-16-phenoxy-17,18,19,20-tetranor-8,13-prostadien.

7. (5Z,13E)-(11R,15R)-1,11,15-Trihydroxy-16,16-dinethyl-5,8,13-prostatrien.

8. (13E)-(11R,15R)=11,15-Dihydroxy-16-phenoxy-17,18,19,20-tetranor-8,13-prostadiensäure-methylsulfonamid.

9. (13E)-(11R,15R)-11,15-Dihydroxy-16,16-dimethyl-8,13-prostadiensäure-methylsulfonamid.

10. (13E)-(11R,15R)-11,15-Dihydroxy-16-phenoxy-17,18,19,20-tetranor-8,13-prostadiensäure-tris(hydroxymethyl) amino-methansalz.

11. (13E)-(11R 15R)-11,15-Dihydroxy-16-phenoxy-17,18,19,20-tetranor-8,13-prostadiensäuremethylester.

12. (5Z,13E)-(11R,16RS)-11,15α-Dihydroxy-16,19-dimethyl-5,8,13,18-prostatetraensäure.

13. (13E)-(11R,16RS)-11,15α-Dihydroxy-16,19-dimethyl-8,13,18-prostatriensäure.

**Claims**

1. $\Delta^{8,9}$-prostane derivatives of the general formula I,

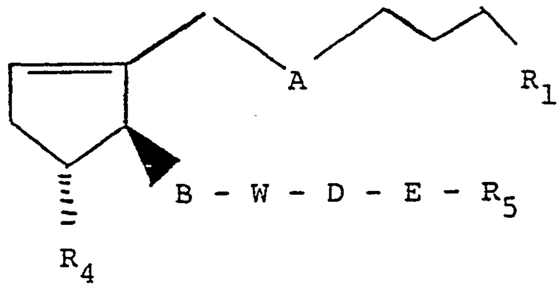

(I)

in which
$R_1$ represents the radical $CH_2OH$ or

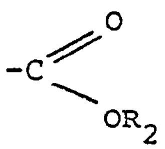

in which $R_2$ denotes a hydrogen atom or a $C_{1-4}$- alkyl group or $R_1$ represents the radical

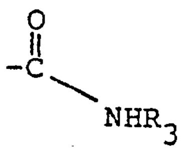

in which $R_3$ denotes a $C_{1-10}$-alkanoyl radical or a methanesulphonyl or isopropanesulphonyl radical or the radical $R_2$, and

A represents a $-CH_2-CH_2-$ or <u>cis</u>-$CH=CH$- group,

B represents a $-CH_2-CH_2-$ group or a <u>trans</u>-$CH=CH$- or a $-C\equiv C-$ group,

W represents a free or functionally modified hydroxymethylene group or a free or functionally modified

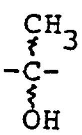

group, it being possible for the OH group to be in the α- or the β-configuration,

D and E together represent a direct bond or

D represents a straight-chain or branched-chain alkylene group having from 1 to 10 carbon atoms which may be optionally substituted by fluorine atoms, and

E represents an oxygen atom or a sulphur atom, a direct bond, a $-C\equiv C-$ bond or a $-CR_6=CR_7-$ group, in which $R_6$ and $R_7$ are different from one another and represent a hydrogen atom, a chlorine atom or an alkyl group having from 1 to 4 carbon atoms,

$R_4$ represents a free or functionally modified hydroxy group,

$R_5$ represents a straight-chain or branched-chain, saturated or unsaturated alkyl group having from 1 to 6 carbon atoms, a benzyl group or, if E represents an oxygen atom, represents a phenyl group;

and, if $R_2$ denotes a hydrogen atom, the salts thereof with physiologically acceptable bases,

and in which the grouping $-DER_5$ does not represent the radical n-pentyl, if W represents a free hydroxymethylene group and if, at the same time, B represents <u>trans</u>-$CH=CH$-, $R_4$ represents OH, A represents <u>cis</u>-$CH=CH$and $R_1$ represents COOH.

2. Process for the manufacture of $\Delta^{8,9}$-prostane derivatives of the general formula I, according to the invention, characterised in that in a manner known <u>per se</u> a compound of the general formula II

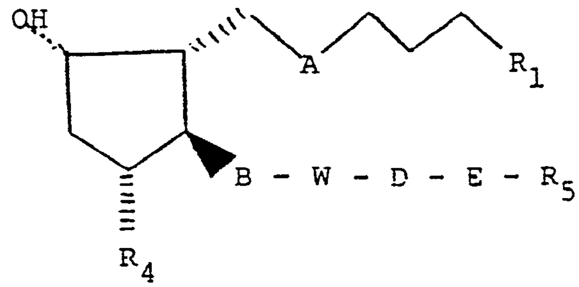

(II),

in which $R_1$ denotes the radicals

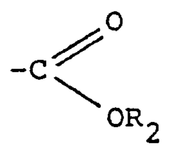

or

or

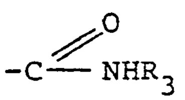

and A, B, D, E and $R_5$ have the meanings given above and free OH groups in $R_4$ and W are protected, is reacted by way of an intermediate 9-sulphonic acid ester with a fluoride in the form of a base and then, optionally, in any desired order, protected hydroxy groups are freed and/or free hydroxy groups are esterified or etherified, and/or double bonds are hydrogenated and/or an esterified carboxy group is hydrolysed and/or a carboxy group is esterified and/or a free carboxy group is converted into an amide and/or a free or esterified carboxy group is reduced and/or a free carboxy group is converted into a salt.

3. Medicaments consisting of one or more compounds according to claim 1 and customary auxiliaries and carriers.

4. (13E)-(11R,15R)-11,15-dihydroxy-16-phenoxy-17,18,19,20-tetranor-8,13-prostadienoic acid.

5. (5Z,13E)-(11R,15R)-11,15-dihydroxy-16,16-dimethyl-5,8,13-prostatrienoic acid.

6. (13E)-(11R,15R)-1,11,15-trihydroxy-16-phenoxy-17,18,19,20-tetranor-8,13-prostadiene.

7. (5Z,13E)-(11R,15R)-1,11,15-trihydroxy-16,16-dimethyl-5,8,13-prostatriene.

8. (13E)-(11R,15R)-11,15-dihydroxy-16-phenoxy-17,18,19,20-tetranor-8,13-prostadienoic acid methylsulfonamide.

9. (13E)-(11R,15R)-11,15-dihydroxy-16,16-dimethyl-8,13-prostadienoic acid methylsulfonamide.

10. The methane salt of (13E)-(11R,15R)-11,15-dihydroxy-16-phenoxy-17,18,19,20-tetranor-8,13-prostadienoic acid tris(hydroxymethyl)-amino.

11. (13E)-(11R,15R)-11,15-dihydroxy-16-phenoxy-17,18,19,20-tetranor-8,13-prostadienoic acid methyl ester.

12. (5Z,13E)-(11R,16RS)-11,15α-dihydroxy-16,19-dimethyl-5,8,13,18-prostatetraenoic acid.

13. (13E)-(11R,16RS)-11,15α-dihydroxy-16 19-dimethyl-8,13,18-prostatrienoic acid.

**Revendications**

1.- Dérivés du Δ $^{8'9}$-prostane qui répondent à la formule générale 1

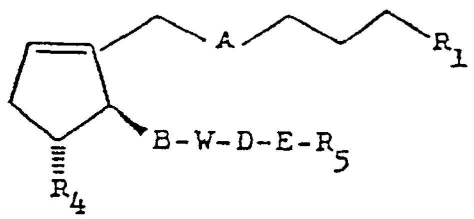

(I)

dans laquelle
$R_1$ représente un radical $-CH_2OH$ un radical

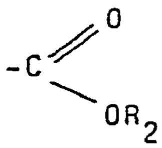

dans lequel $R_2$ désigne un atome d'hydrogène ou un alkyle en $C_1$-$C_4$, ou $R_1$ représente un radical

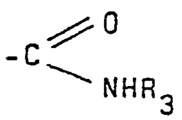

dans lequel $R_3$ désigne un alcanoyle en $C_1$-$C_{10}$, un méthane-sulfonyle ou un (méthyl-1 éthane)-sulfonyle ou a la signification donnée pour $R_2$,

A représente un radical $-CH_2-CH_2-$ ou un radical $-CH=CH-$ cis.

B représente un radical $-CH_2-CH_2-$, un radical $-CH=CH$trans ou un radical $-C\equiv C-$,

W représente un radical hydroxy-méthylène libre ou sous la forme d'un dérivé fonctionnel, ou un radical

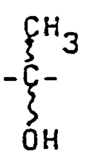

libre ou sous la forme d'un dérivé fonctionnel, le radical -OH ayant la configuration,α ou la configuration β,

D et E forment ensemble une liaison directe ou

D représente un radical alkylène, linéaire ou ramifié, qui contient de 1 à 10 atomes de carbone et qui peut porter des atomes de fluor,

E représente un atome d'oxygène ou de soufre, une liaison directe, un radical $-C\equiv C-$ ou un radical $-CR_6=CR_7-$ dans lequel $R_6$ et $R_7$ sont différents l'un de l'autre et représentent chacun un atome d'hydrogène ou de chlore ou un alkyle en $C_1$-$C_4$,

$R_4$ représente un radical hydroxy libre ou sous la forme d'un dérivé fonctionnel, et

$R_5$ représente un radical alkyle linéaire ou ramifié, saturé ou insaturé qui contient de 1 à 6 atomes de carbone, un radical benzyle ou, lorsque E désigne un atome d'oxygène, un radical phényle, avec la condition que l'ensemble $-OER_5$ ne represente pas un radical n-pentyle dans le cas où, en méme temps, W désigne un radical hydroxy-méthylène libre, B un radical $-CH=CH-$ trans, $R_4$ un radical -OH, A un radical $-CH=CH-$ cis et $R_1$ un radical -COOH, ainsi que les sels qu'ils forment, lorsque $R_2$ désigne un atome d'hydrogène, avec des bases acceptables du point de vue physiologique.

2.- Procédé pour préparer les dérivés du $\Delta^{8,9}$-prostane de formule générale I conformes à 1,in-vention, procédé caractérisé en ce que, en opérant de manière connue, on fait réagir un composé répondant à la formule générale II :

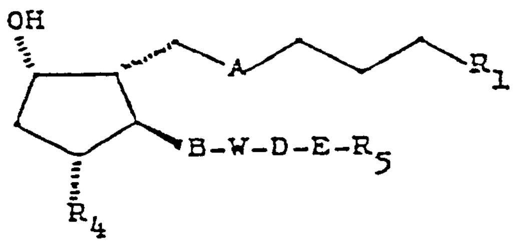

(II)

dans laquelle $R_1$ représente un radical

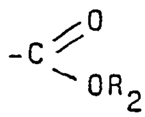

ou

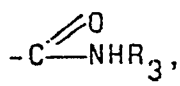,

les symboles A, B, D, E et $R_5$ ont les significations indiquées ci-dessus, et les radicaux hydroxy dans $R_4$ et W sont protégés, par l'intermédiaire d'un ester sulfonique en 9, avec un fluorure en tant que base, puis on effectue éventuellement, dans l'ordre que l'on veut, les réactions suivantes : on libère des radicaux hydroxy protégés et/ou on estérifie ou éthérifie des radicaux hydroxy libres et/ou on hydrogène des doubles liaisons et/ou on saponifie un radical carboxy estérifié et/ou on estérifie un radical carboxy et/ou on transforme un radical carboxy libre en un radical carbamoyle et/ou on réduit un radical carboxy libre ou estérifié et/ou on transforme en un sel un radical carboxy libre.

3.- Médicament consistant en un ou plusieurs composés selon la revendication 1 et en excipients et adjuvants usuels.

4.- Acide dihydroxy-11,15 phénoxy-16 tétranor-17,18,19,20 prostadiène-8,13 oïque-(13E)-(11R,15R).

5.- Acide dihydroxy-11,15 diméthyl-16,16 prostatriène-5,9,13 oïque-(5Z,13E)-(11R,i5R).

6.- Trihydroxy-1,11,15 phénoxy-16 tétranor-17,18,19,20 prostadiène-8,13 (13E)-(11R,15R).

7.- Trihydroxy-1,11,15 diméthyl-16,16 prostatriène-5,8,13 (5Z,13E)-(11R,15R)

8.- N-Méthylsulfonyl-amide de l'acide dihydroxy-11,15 phénoxy-16 tétranor-17,18,19,20 prosta-diène-8,13 oïque-(13E)-(11R 15R)

9.- N-Méthylsulfonyl-amide de l'acide dihydroxy-11,15 diméthyl-16,16 prostadiène-8,13 oïque-(13E)-(11R,15R).

10.- Sel formé par l'acide dihydroxy-11,15 phénoxy-16 tétranor-17,18,19,20 prostadiène-8,13 oïque-(13E)-(11R,15R) avec la tris-(hydroxyméthyl)-méthylamine.

11.- Ester méthylique de l'acide dihydroxy-11,15 phénoxy-16 tétranor-17,18,19,20 prosta-diène-8,13 oïque-(13E)-(11R,15R).

12.- Acide dihydroxy-11,15 α diméthyl-16,19 prostatétraène-5,8,13,19 oïque-(5Z,13E)-(11R,16RS).

13.- Acide dihydroxy-11 15 α diméthyl-16,19 prostatriène-8,13,18 oïque-(13E)-(11R 16RS).